# EUROPÄISCHE PATENTSCHRIFT

(11) **EP 3 457 110 B1**
(45) Veröffentlichungstag und Bekanntmachung des Hinweises auf die Patenterteilung: **06.11.2024**
(21) Anmeldenummer: 18194235.0
(22) Anmeldetag: 13.09.2018
(51) Int. Cl.: G01N 1/34, G01N 1/38, G01N 1/40, G01N 33/20

(54) **VERFAHREN ZUR ANALYSE VON PARTIKELN IN STAHL, INSBESONDERE VON AUSSCHEIDUNGEN ODER/UND EINSCHLÜSSEN**
METHOD FOR ANALYSING PARTICLES IN STEEL, IN PARTICULAR DEPOSIT FORMATION OR/AND INCLUSIONS
PROCÉDÉ D'ANALYSE DES PARTICULES EN ACIER, EN PARTICULIER DE SÉPARATIONS ET / OU D'INCLUSION

(30) Priorität: 18.09.2017 DE 102017121595
(43) Veröffentlichungstag der Anmeldung: 20.03.2019
(73) Patentinhaber: Aktiengesellschaft der Dillinger Hüttenwerke, 66763 Dillingen/Saar (DE)
(72) Erfinder: Staudt, Thorsten, 66839 Schmelz-Hüttersdorf (DE); Hegetschweiler, Andreas, 66386 St.Ingbert (DE); Kraus, Tobias, 66111 Saarbrücken (DE)
(74) Vertreter: Patentanwälte Bernhardt / Wolff Partnerschaft mbB

(56) Entgegenhaltungen:
- EP-A1- 2 270 469
- EP-A1- 2 624 812
- EP-A1- 3 418 712
- JP-A- 2009 008 584
- JP-A- H05 346 387
- US-A1- 2010 206 736
- US-B2- 7 828 999
- MÜLLER A. ET AL: "Structure formation of surfactants in concentrated sulphuric acid: a light scattering study", COLLOID & POLYMER SCIENCE, vol. 273, no. 9, 1 September 1995 (1995-09-01), DE, pages 866 - 875, XP055868231, ISSN: 0303-402X, Retrieved from the Internet <URL:http://link.springer.com/article/10.1007/BF00657636/fulltext.html> [retrieved on 20211201], DOI: 10.1007/BF00657636

## Beschreibung

Die Erfindung betrifft ein Verfahren zur Analyse von Partikeln in Stahl, insbesondere von Ausscheidungen oder/und Einschlüssen, bei dem die Partikel mittels eines Extraktionsmittels, das eine Säure enthält, aus dem Stahl extrahiert werden und danach analysiert werden.

Ein Analyseverfahren für Stahlpartikel geht aus der EP 2 270 469 A1 hervor. Partikel, die in einem zu analysierenden metallischen Material enthalten sind, werden in einem Lösungsmittel extrahiert, die beim Extrahieren isolierten Partikel danach vollständig von dem Lösungsmittel getrennt und zur Herstellung einer Dispersion in ein organisches Lösungsmittel gegeben. Danach wird die Dispersion mittels Feld-Fluss-Fraktionierung analysiert.

Ein weiteres Partikelanalyseverfahren ist aus der JP H05 346387 A bekannt. Unter Verwendung einer Säure werden Partikel aus einem Stahl herausgelöst und anschließend die herausgelösten Partikel in einem Dispergiermedium dispergiert. Zur anschließenden Analyse werden beispielsweise Verfahren der Lichtstreuung eingesetzt.

Aus der JP 2009 008584 A geht ein Verfahren zur Analyse von Partikeln in Stahl hervor, bei welchem zum Herauslösen der Partikel eine Lösung mit Eigenschaften eines Dispergiermittels verwendet wird. Die herausgelösten Partikel werden mittels dynamischer Lichtstreuung untersucht.

EP 3 418 712 A1 beschreibt ein Verfahren zur Partikelanalyse, bei dem Partikel unter Verwendung einer Elektrolyse aus einem metallischen Material gelöst werden, wobei die hierbei verwendete Lösung ein Dispergiermittel enthalten kann. Nach dem Herauslösen werden die hierbei erhaltenen Partikel mittels energiedispersiver Röntgenspektroskopie analysiert.

Aus der US 2010/206736 A1 ist ein Verfahren zur Analyse von Partikeln in Metall bekannt, bei dem mittels Elektrolyse die Partikel extrahiert und nach der Entfernung des Elektrolyts zur Herstellung einer Dispersion in ein Lösungsmittel gegeben werden. Anschließend werden die Partikel in der Dispersion analysiert.

Der Erfindung liegt die Aufgabe zugrunde, ein Analyseverfahren der eingangs genannten Art zu schaffen, das zuverlässigere Ergebnisse liefert.

Erfindungsgemäß wird diese Aufgabe dadurch gelöst, dass das Extraktionsmittel zur Vermeidung einer Agglomeration der Partikel mit einem Dispergiermittel versehen wird, das ein Copolymer ausgewählt aus der Gruppe bestehend aus Acrylat-Copolymer, Meth(acryl-) basiertem Copolymer, Meth(acryl-) basiertem Terpolymer, Maleinsäure basiertem Copolymer, Maleinsäure basiertem Terpolymer, p-tert-Octylphenol-Derivat mit Polyethylenglycolseitenkette (auch bekannt unter den Markennamen "Triton X-100" und "Nonidet P40"), Sorbitanfettsäureester, Polyoxyethylensorbitanfettsäurester, Poly(3,4-Dihydroxyphenylalanin)-Polylactid Copolymer und Poly(3,4-Dihydroxyphenylalanin)-Polyethylenglycol Copolymer enthält.

Der Stahl wird durch das Extraktionsmittel zunächst so zersetzt, dass sich eine Suspension aus den extrahierten Partikeln und dem Extraktionsmittel, das mit durch die Extraktion zersetzten Komponenten des Stahls beladen ist, bildet. Das Dispergiermittel ordnet sich an der Oberfläche der Partikel an und stabilisiert sie gegen Agglomerierung miteinander dadurch, dass sich die mit dem Dispergiermittel belegten Oberflächen gegenseitig abstoßen. Eine Agglomeration der Partikel, die dadurch verstärkt werden kann, dass sich beim Extrahieren in der sich bildenden Suspension ein SiO₂-Netzwerk von aus dem Stahl extrahierten Silicium bildet, das die Partikel umschließt, wird so schon während der Extraktion vermieden. Dies wirkt sich vorteilhaft auf die Qualität der Analyse, insbesondere einer Messung der Partikelgrößen und/oder der Partikelgrößenverteilung, auf, da Partikelagglomerationen ein Analyseergebnis erheblich verändern können.

Es hat sich überraschend gezeigt, dass das Dispergiermittel dadurch, dass es sich an der Partikeloberfläche anordnet, darüber hinaus vermeidet, dass die Partikel durch das Extraktionsmittel zersetzt werden. Auch dieser Effekt führt zu zuverlässigeren Ergebnissen, da schon eine nur geringfügige Zersetzung der Partikel die Größen der Partikel verändert und die Analyse folglich verfälschen kann.

Ferner lässt sich das erfindungsgemäße Verfahren im Vergleich zu demjenigen nach dem Stand der Technik einfacher durchführen, da eine vollständige Trennung der extrahierten Partikel von dem Extraktionsmittel, insbesondere ein vollständiger Austausch der Flüssigkeit, in der die Partikel angeordnet sind, zur Durchführung der Analyse nicht notwendig ist.

Das Verfahren hat sich als besonders vorteilhaft zur Analyse von Ausscheidungen in mikrolegiertem Stahl erwiesen. Die Partikel, die sich mittels des Verfahrens analysieren lassen, enthalten vorzugsweise die Legierungselemente Titan, Niob, Aluminium und/oder Vanadium, vorzugsweise Titannitrid, Titancarbid, Titancarbonitrid, Niobcarbid, Niobcarbonitrid und/oder Carbide, Nitride oder Carbonitride, die Titan und Niob aufweisen.

Die Extraktion wird zweckmäßigerweise an Stahlspäne durchgeführt.

Während es vorstellbar wäre, ein elektrostatisch oder ein elektrosterisch stabilisierend wirkendes Dispergiermittel zu verwenden, wird in einer bevorzugten Ausführungsform der Erfindung ein sterisch stabilisierendes Dispergiermittel benutzt.

Vorzugsweise ist das sterische Dispergiermittel eine Lösung eines Tensids mit einem Molekülteil, der eine vergleichsweise große Affinität zu den Partikeln aufweist und sich dort anordnet, und einem anderen Molekülteil, der von den Partikeln wegsteht und die Partikel im Abstand voneinander hält. Zweckmäßigerweise wird das sterische Dispergiermittel in dem Extraktionsmittel in einer Konzentration von 0,05 bis 0,5 Vol.-%, vorzugsweise 0,08 bis 0,15 Vol.-%, vorgesehen.

In einer Ausgestaltung der Erfindung enthält das Dispergiermittel einen Stoff ausgewählt aus der Gruppe bestehend aus Copolymer, anionischem Polymer, kationischem Polymer und nicht-ionischem Polymer.

Als den Sorbitanfettsäureester kann das Dispergiermittel z. B. Sorbitanmonolaurat (auch bekannt unter dem Markennamen "Span 20") und als den PolyoxyethylenSorbitanfettsäurester beispielsweise Polyoxyethylen(20)-Sorbitan-Monostearat (auch bekannt unter dem Markennamen "Tween 60") enthalten.

In einer bevorzugten Ausführungsform der Erfindung enthält das Copolymer langkettige, verzweigte Akylketten mit konjugierten Ketongruppen. Zweckmäßigerweise weist das Copolymer funktionelle Gruppen ausgewählt aus der Gruppe bestehen aus Ether-, Carboxyl-, Nitro-, Hydroxyl-, Amino-, Arylthioether- und Estergruppen und Heterocyclen, vorzugsweise Pyridin oder/und Thiophen, auf.

In einer weiteren Ausführungsform der Erfindung enthält das Dispergiermittel ein anionisches Polymer ausgewählt aus der Gruppe bestehend aus Polystyrolsulfonat, Fettalkoholsulfat, Alkylethersulfat, Alkylbenzolsulfonat, Alkylcarboxylat, Alkylaryletherphosphat und Alkyletherphosphat. Als das Fettalkoholsulfat kann das Dispergiermittel beispielsweise Natriumlaurylsulfat, als das Alkylethersulfat Natrium-dodecylpoly(oxyethylen)sulfat und als das Alkylbenzolsulfonat Natriumdodecylbenuzolsulfonat enthalten.

Ferner könnte das Disperigermittel ein kationisches Polymer ausgewählt aus der Gruppe bestehend aus Polyethylenimin (PEI) und einer Alkylammoniumverbindung, vorzugsweise Cetyltrimethylammoniumbromid (CTAB), enthalten.

In einer weiteren Ausführungsform der Erfindung enthält das Dispergiermittel ein nichtionisches Polymer ausgewählt aus der Gruppe bestehend aus Polyoxyethylen, Polyamin und Poly(meth)acrylat.

In einer Ausführungsform der Erfindung ist das Dispergiermittel vorgesehen derart, dass es sich auch an der Oberfläche von SiO₂-Teilchen anordnen kann und so auch die Oberflächen der SiO₂-Teilchen in der Suspension stabilisiert, um die oben genannte SiO₂-Netzwerkbildung zu vermeiden. Zweckmäßigerweise weist das Tensid einen Bestandteil, insbesondere einen Molekülteil, auf, der mit dem SiO₂ wechselwirkt und vorzugsweise eine vergleichsweise große Affinität dazu aufweist.

Während es vorstellbar wäre, das Dispergiermittel während dem Extrahieren zu dem Extraktionsmittel zu geben, wird es bevorzugt vor der Extraktion zu dem Extraktionsmittel hinzugefügt.

Zweckmäßigerweise ist das Extraktionsmittel eine wässrige Lösung.

Vorstellbar wäre aber auch, eine organische Lösung für die Extraktion zu benutzen.

Das Extraktionsmittel enthält zum Zersetzen des Stahls eine Säure, vorzugsweise Salzsäure oder Schwefelsäure. Die Säure wird zweckmäßigerweise in einer derartigen Konzentration vorgesehen, dass die Partikel von der Säure nicht oder nur geringfügig angegriffen werden, um zu vermeiden, dass die Analyse durch eine Zersetzung der Partikel beeinflusst wird.

Es hat sich als geeignet erwiesen, die Salzsäure in einer Konzentration von 2 bis 6 mol/l, vorzugsweise 3 bis 4 mol/l, oder die Schwefelsäure in einer Konzentration von 0,5 bis 3 mol/l, vorzugsweise 1,5 bis 2,5 mol/l, in dem Extraktionsmittel, insbesondere der wässrigen Lösung, vorzusehen.

Zweckmäßigerweise wird die Extraktion bei einer Temperatur > 50 °C, vorzugsweise > 65 °C, durchgeführt. Die Extraktion bei diesen Temperaturen hat sich als vorteilhaft erwiesen, weil die Zersetzung des Stahls dann schneller abläuft als bei Raumtemperatur. Da die genannte Bildung der SiO₂-Netzwerke umso stärker wird, je länger die Extraktion dauert, kann durch einen schnellen Ablauf der Extraktion die Agglomeration der Partikel noch besser vermieden werden.

In einer weiteren Ausführungsform der Erfindung wird die Suspension, die sich durch die Extraktion aus dem beladenen Extraktionsmittel und den Partikeln gebildet hat, einem Trennverfahren, vorzugsweise mittels einer Zentrifuge oder/und Sedimentation, unterzogen. Zweckmäßigerweise wird bei Durchführung des Trennverfahrens das beladene Extraktionsmittel zumindest teilweise von den Partikeln getrennt.

Nach der teilweisen Trennung des beladenen Extraktionsmittels von den Partikeln kann zu einer Restsuspension aus dem nach der Trennung verbleibenden Extraktionsmittel und den Partikeln ein Lösungsmittel, vorzugsweise destilliertes Wasser oder ein organisches Lösungsmittel, gegeben werden, wobei dafür besonders bevorzugt dasselbe Lösungsmittel verwendet wird, das das Extraktionsmittel aufweist.

Es hat sich als vorteilhaft erwiesen, das Trennverfahren mehrfach durchzuführen, ggf. einschließlich der Zugabe des Lösungsmittels, um zu vermeiden, dass aus der Extraktion verbleibende Bestandteile des Stahls das Analyseergebnis beeinflussen. Zweckmäßigerweise wird das Trennverfahren derart durchgeführt, dass das Dispergiermittel, das sich bei der Extraktion auf den Partikeln angeordnet hat, zur Vermeidung der Bildung von Agglomeraten auch nach Durchführung des Trennverfahrens zur Stabilisierung auf den Partikeln verbleibt.

Die Suspension, die sich durch die Extraktion aus dem beladenen Extraktionsmittel und den Partikeln gebildet hat, wird vorzugsweise während oder/und nach Durchführung des Trennverfahrens, ggf. nach Zugabe des Lösungsmittels, dispergiert, um ein Agglomerieren der Partikel während des Trennverfahrens und/oder danach zu vermeiden.

In einer Ausgestaltung der Erfindung wird die Analyse der Partikel, insbesondere die Messung der Partikelgrößen oder der Partikelgrößenverteilung, an der unmittelbar aus dem Trennverfahren erhaltenen Suspension durchgeführt oder die Suspension vor der Analyse mit einem Lösungsmittel, ggf. unter Dispergierung, vermischt.

Die Partikel können beispielsweise mittels dynamischer Lichtstreuung, asymmetrischer Feld-Fluss-Fraktionierung oder/und mittels einer analytischen Ultrazentrifuge analysiert werden.

Sofern die Partikel mittels eines Analyseverfahrens wie Transmissionselektronenmikroskopie (TEM) oder Raster-Transmissionselektronenmikroskopie (STEM), das nicht an der Suspension durchgeführt werden kann, analysiert werden, wird zweckmäßigerweise eine Probe analysiert, die unmittelbar aus der Suspension, vorzugsweise durch Trocknung, hergestellt worden ist.

Die Erfindung wird nachfolgend anhand von Beispielen und der beigefügten Abbildungen weiter erläutert. Es zeigen:
- Fig. 1: TEM-Aufnahmen von Proben, und
- Fig. 2: Messergebnisse zu den Proben.

### 1. Beispiel:

### 1. Extraktion:

1 g Späne aus mikrolegiertem Stahl wird in 50 ml Extraktionsmittel extrahiert, das eine wässrige Schwefelsäurelösung mit 0,5 mol/l Schwefelsäure enthält und mit 0,1 Vol.-% eines Dispergiermittels, das ein strukturiertes Acrylat-Copolymer mit Pigmentaffinen Gruppen, die Carbonyl-, Amino- und Estergruppen sowie Pyridin und Thioplan umfassen, ist. Das Dispergiermittel wirkt sterisch stabilisierend und ist eine Lösung eines Tensids mit einer Molekülgruppe, die affin zu Titannitrid, Titancarbid, Titancarbonitrid, Niobcarbid, Niobcarbonitrid und/oder Carbid, Nitrid oder Carbonitrid, das Titan und Niob aufweist, ist. Die Extraktion wird bei 70 °C in einem Ölbad durchgeführt. Die Späne verbleibt so lange in dem Extraktionsmittel, bis die Stahlmatrix des Stahls zersetzt ist und die aus den Ausscheidungen gebildeten Partikel freigegeben sind. Bei der Extraktion belegt das Dispergiermittel die Oberflächen der Partikel.

### 2. Trennverfahren:

Nach der Extraktion wird eine Suspension, die sich aus dem Extraktionsmittel, das mit der zersetzten Stahlmatrix beladen ist, und den belegten Partikeln bildet, mit einer Ultrazentrifuge bearbeitet. Die Partikel sedimentieren dabei am Boden Flüssigkeit der Suspension, die oberhalb eines Bodensatzes aus den Partikeln verbleibt, wird entfernt und durch destilliertes Wasser ersetzt. Anschließend wird die so gebildete Restsuspension dispergiert. Um die Bestandteile des zersetzten Stahls größtenteils aus der Restsuspension zu entfernen, wird viermal zentrifugiert und sukzessive der jeweils oberhalb des Bodensatzes verbleibende Teil der Flüssigkeit der jeweilig gebildeten Restsuspension entfernt.

### 3. Analyse:

Die nach dem Trennverfahren erhaltene Restsuspension wird auf einem Probenträger getrocknet und die erhaltene Probe aus den Partikeln mittels Transmissionselektronenmikroskopie (TEM) analysiert. Zum einen wird die Partikelgrößenverteilung mittels einer geeigneten Auswertesoftware bestimmt, zum anderen kann mittels energiegefilterter Transmissionselektronenmikroskopie (EFTEM) die chemische Zusammensetzung der Partikel ermittelt werden.

Es hat sich gezeigt, dass die Partikel in den hergestellten Proben gut erkennbar und nicht agglomeriert sind, sodass die Messung der Partikelgrößenverteilung sehr genaue Ergebnisse liefert.

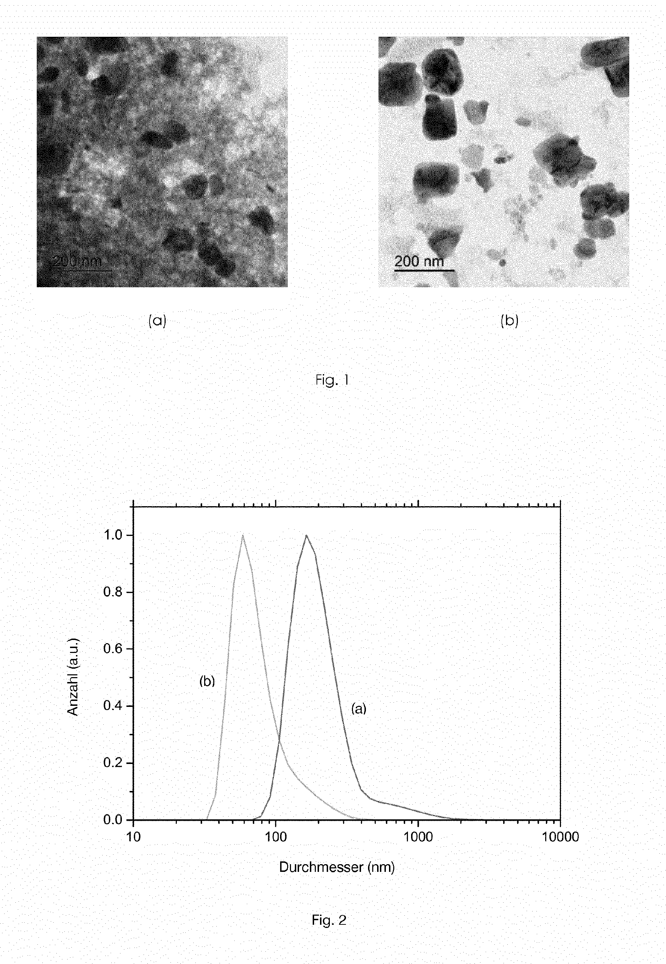

Fig. 1 zeigt unter (a) eine mittels TEM abgebildete Probe, bei deren Herstellung zu dem Extraktionsmittel während der Extraktion kein Dispergiermittel gegeben worden ist, und unter (b) eine andere Probe, bei deren Herstellung das Extraktionsmittel bereits vor der Extraktion mit dem Dispergiermittel vermischt worden ist. Beim Vergleich der Abbildungen zeigt sich, dass die Partikel der Probe nach Fig. 1 (b) wesentlich weniger agglomeriert sind als diejenigen nach Fig. 1 (a). In der TEM-Aufnahme in Fig. 1 (a) ist zwischen den größeren, dunklen Partikeln, erkennbar, dass sich ein SiO₂-Netzwerk gebildet hat. Dieses findet sich wegen der Verwendung des Dispergiermittels bei der in Fig. 1 (b) abgebildeten Probe nicht.

Fig. 2 zeigt Messungen der Proben mittels dynamischer Lichtstreuung. Bei der Probe (b), zu deren Herstellung wie oben erläutert das Dispergiermittel benutzt worden ist, sind wesentlich kleinere Partikeldurchmesser gemessen worden als bei der Probe (a), die nicht unter Benutzung des Dispergiermittels hergestellt worden ist. Auch wenn bei der Probe (b) tatsächlich aufgrund von Messungenauigkeiten möglicherweise noch kleinere Partikeldurchmesser vorliegen als das Messergebnis zeigt, konnten durch die Benutzung des Dispergiermittels Partikeldurchmesser ermittelt werden, die den tatsächlichen Größen der Partikel im Stahl entsprechen. Folglich belegen die Messergebnisse die positive Wirkung der Verwendung des Dispergiermittels zur Vermeidung der Bildung von Agglomeraten.

Alternativ könnte eine Analyse auch mittels Raster-Transmissionselektronenmikroskopie (STEM) durchgeführt werden.

Ferner könnte die nach dem Trennverfahren erhaltene Restsuspension auch mittels dynamischer Lichtstreuung, asymmetrischer Fluss Feld-Fluss Fraktionierung oder einer analytischen Ultrazentrifuge analysiert werden.

Bei der asymmetrischen Fluss Feld-Fluss Fraktionierung (AF4, A4F, auch FFF) mit statischer Lichtstreuung (MALS) und UV-Vis Detektor wird die Partikelsuspension zunächst in der Feld-Fluss-Fraktionierung in verschiedene Größenfraktionen aufgetrennt und anschließend analysiert. Durch die Auftrennung wird eine gute Auflösung erreicht.

Bei Verwendung der analytische Ultrazentrifuge wird die Sedimentation der Partikel in einem Schwerefeld mittels optischer Spektroskopie betrachtet. Die Partikel werden dabei während der Analyse nach ihrer Größe aufgetrennt. Aus dem ermittelten Sedimentationskoeffizienten kann der hydrodynamische Radius bestimmt werden und daraus die Partikelgrößenverteilung ermittelt werden.

### 2. Beispiel:

Abweichend vom 1. Beispiel wird die Extraktion in 50 ml wässriger Säurelösung, die 3 mol/l Salzsäure und 0,3 Vol.-% des Dispergiermittels, das oben unter Beispiel 1 beschrieben ist, enthält, durchgeführt. Sonstige Verfahrensschritte werden wie im 1. Beispiel durchgeführt.

### 3. Beispiel:

Abweichend vom 1. Beispiel wird die Extraktion in 50 ml Säurelösung, die 1,8 mol/l Schwefelsäure und 0,3 Vol.-% Polystyrolsulfonat als Dispergiermittel enthält, durchgeführt. Sonstige Verfahrensschritte werden wie im 1. Beispiel durchgeführt.

## Patentansprüche

1. Verfahren zur Analyse von Partikeln in Stahl, insbesondere von Ausscheidungen oder/und Einschlüssen, bei dem die Partikel mittels eines Extraktionsmittels, das eine Säure enthält, aus dem Stahl extrahiert werden und danach analysiert werden,
**dadurch gekennzeichnet,**
**dass** das Extraktionsmittel zur Vermeidung einer Agglomeration der Partikel mit einem Dispergiermittel versehen wird, das ein Copolymer ausgewählt aus der Gruppe bestehend aus Acrylat-Copolymer, Meth(acryl-) basiertem Copolymer oder Terpolymer, Maleinsäure basiertem Copolymer oder Terpolymer, p-tert-Octylphenol-Derivat mit Polyethylenglycolseitenkette, Sorbitanfettsäureester, Polyoxyethylensorbitanfettsäurester, Poly(3,4-Dihydroxyphenylalanin)-Polylactid Copolymer und Poly(3,4-Dihydroxyphenylalanin)-Polyethylenglycol Copolymer enthält.

2. Verfahren nach Anspruch 1,
**dadurch gekennzeichnet,**
**dass** das Extraktionsmittel eine wässrige Lösung ist.

3. Verfahren nach Anspruch 1 oder 2,
**dadurch gekennzeichnet,**
**dass** das Extraktionsmittel Salzsäure oder Schwefelsäure enthält.

4. Verfahren nach Anspruch 3,
**dadurch gekennzeichnet,**
**dass** das Extraktionsmittel die Salzsäure in einer Konzentration von 1,5 bis 6 mol/l, vorzugsweise 1,8 bis 4 mol/l, oder die Schwefelsäure in einer Konzentration von 0,1 bis 3 mol/l, vorzugsweise 0,3 bis 1 mol/l, enthält.

5. Verfahren nach einem der Ansprüche 1 bis 4,
**dadurch gekennzeichnet,**
**dass** eine Suspension, die sich durch die Extraktion aus dem mit Bestandteilen des zersetzten Stahls beladenen Extraktionsmittel und den Partikeln gebildet hat, ggf. mehrfach, einem Trennverfahren, vorzugsweise Zentrifugieren oder/und Sedimentieren, unterzogen wird.

6. Verfahren nach Anspruch 5,
**dadurch gekennzeichnet,**
**dass** bei Durchführung des Trennverfahrens das beladene Extraktionsmittel zumindest teilweise von den Partikeln getrennt wird.

7. Verfahren nach Anspruch 6,
**dadurch gekennzeichnet,**
**dass** nach der teilweisen Trennung des beladenen Extraktionsmittels von den Partikeln in eine Restsuspension, die aus dem nach der Trennung verbleibenden Extraktionsmittel und den Partikeln gebildet ist, ein Lösungsmittel, vorzugsweise Wasser, gegeben wird.

8. Verfahren nach einem der Ansprüche 5 bis 7,
**dadurch gekennzeichnet,**
**dass** das Trennverfahren derart durchgeführt wird, dass das Dispergiermittel, das sich bei der Extraktion auf den Partikeln angeordnet hat, zur Stabilisierung der Suspension auf den Partikeln verbleibt.

9. Verfahren nach einem der Ansprüche 5 bis 8,
**dadurch gekennzeichnet,**
**dass** eine Analyse der Partikel, insbesondere eine Messung der Partikelgrößen oder Partikelgrößenverteilung, direkt an der nach dem Trennverfahren erhaltenen Suspension oder an einer Probe durchgeführt wird, die unmittelbar aus der Suspension, vorzugsweise durch Trocknung, hergestellt worden ist.

10. Verfahren nach einem der Ansprüche 1 bis 9,
**dadurch gekennzeichnet,**
**dass** die Partikel mittels dynamischer Lichtstreuung, asymmetrischer Feld-Fluss-Fraktionierung, mittels einer analytischen Ultrazentrifuge oder/und mittels Elektronenmikroskopie, vorzugsweise Transmissionselektronenmikroskopie (TEM) oder Raster-Transmissionselektronenmikroskopie (STEM), analysiert werden.

11. Verfahren nach einem der Ansprüche 1 bis 10,
**dadurch gekennzeichnet,**
**dass** der Stahl ein mikrolegierter Stahl ist und die Partikel die Legierungselemente Titan, Niob, Aluminium und/oder Vanadium, vorzugsweise Titannitrid, Titancarbid, Titancarbonitrid, Niobcarbid, Niobcarbonitrid, und/oder Carbide, Nitride oder Carbonitride, das Titan und Niob aufweist, enthalten.

12. Suspension, die ein Mittel zur Extraktion von Partikeln, insbesondere Ausscheidungen oder/und Einschlüssen, aus Stahl, das eine den Stahl zersetzende Säure enthält, aus Stahl extrahierte Partikel und durch die Extraktion zersetzte Komponenten des Stahls enthält,
**dadurch gekennzeichnet,**
**dass** das Extraktionsmittel zur Vermeidung einer Agglomeration der Partikel ein Dispergiermittel enthält, das ein Copolymer ausgewdhlt aus der Gruppe bestehend aus Acrylat-Copolymer, Meth(acryl-) basiertem Copolymer oder Terpolymer, Maleinsäure basiertem Copolymer oder Terpolymer, p-tert-Octylphenol-Derivat mit Polyethylenglycolseitenkette, Sorbitanfettsäureester, Polyoxyethylensorbitanfettsäurester, Poly(3,4-Dihydroxyphenylalanin)-Polylactid Copolymer und Poly(3,4-Dihydroxyphenylalanin)-Polyethylenglycol Copolymer enthält.

## Claims

1. Method for analysing particles in steel, especially precipitates and/or inclusions, wherein the particles are extracted from the steel by means of an extraction medium containing an acid and are thereafter analysed, **characterized**
**in that** to prevent agglomeration of the particles, the extraction medium is provided with a dispersant containing a copolymer selected from the group consisting of acrylate copolymer, meth(acrylic)-based copolymer or terpolymer, maleic acid-based copolymer or terpolymer, p-tert-octylphenol derivative with polyethylene glycol side chain, sorbitan fatty acid ester, polyoxyethylene sorbitan fatty acid ester, poly(3,4-dihydroxy-phenylalanine)-polylactide copolymer and poly(3,4-dihydroxyphenylalanine)-polyethylene glycol copolymer.

2. Method according to Claim 1,
**characterized**
**in that** the extraction medium is an aqueous solution.

3. Method according to Claim 1 or 2,
**characterized**
**in that** the extraction medium contains hydrochloric acid or sulfuric acid.

4. Method according to Claim 3,
**characterized**
**in that** the extraction medium contains the hydrochloric acid in a concentration of 1.5 to 6 mol/l, preferably 1.8 to 4 mol/l, or contains the sulfuric acid in a concentration of 0.1 to 3 mol/l, preferably 0.3 to 1 mol/l.

5. Method according to any of Claims 1 to 4, **characterized**
**in that** a suspension formed by the extraction, composed of the particles and the extraction medium laden with constituents of the decomposed steel, is subjected, optionally two or more times, to a separation method, preferably centrifugation and/or sedimentation.

6. Method according to Claim 5,
**characterized**
**in that** when the separation method is carried out, the laden extraction medium is separated at least partly from the particles.

7. Method according to Claim 6,
**characterized**
**in that** after the partial separation of the laden extraction medium from the particles, a solvent, preferably water, is added to a residual suspension formed of the particles and the extraction medium remaining after the separation.

8. Method according to any of Claims 5 to 7, **characterized**
**in that** the separation method is carried out in such a way that the dispersant which has attached to the particles during the extraction remains on the particles to stabilize the suspension.

9. Method according to any of Claims 5 to 8, **characterized**
**in that** an analysis of the particles, more particularly a measurement of the particle sizes or particle size distribution, is carried out directly on the suspension obtained after the separation method or on a sample produced directly from the suspension, preferably by drying.

10. Method according to any of Claims 1 to 9, **characterized**
**in that** the particles are analysed by means of dynamic light scattering, asymmetric field flow fractionation, by means of an analytical ultracentrifuge and/or by means of electron microscopy, preferably transmission electron microscopy (TEM) or scanning transmission electron microscopy (STEM).

11. Method according to any of Claims 1 to 10, **characterized**
**in that** the steel is a microalloyed steel and the particles contain the alloy elements titanium, niobium, aluminium and/or vanadium, preferably titanium nitride, titanium carbide, titanium carbonitride, niobium carbide, niobium carbonitride, and/or carbides, nitrides or carbonitrides comprising titanium and niobium.

12. Suspension containing a medium for the extraction of particles, more particularly precipitates and/or inclusions, from steel, containing an acid which decomposes the steel, particles extracted from steel and components of the steel decomposed as a result of the extraction,
**characterized**
**in that** to prevent agglomeration of the particles, the extraction medium contains a dispersant containing a copolymer selected from the group consisting of acrylate copolymer, meth(acrylic)-based copolymer or terpolymer, maleic acid-based copolymer or terpolymer, p-tert-octylphenol derivative with polyethylene glycol side chain, sorbitan fatty acid ester, polyoxyethylene sorbitan fatty acid ester, poly(3,4-dihydroxy-phenylalanine)-polylactide copolymer and poly(3,4-dihydroxyphenylalanine)-polyethylene glycol copolymer.

## Revendications

1. Procédé d'analyse de particules dans l'acier, en particulier de précipités ou/et d'inclusions, dans lequel les particules sont extraites de l'acier à l'aide d'un agent d'extraction contenant de l'acide, et sont ensuite analysées, **caractérisé en ce que**
pour éviter l'agglomération des particules, l'agent d'extraction est pourvu d'un agent dispersant qui contient un copolymère choisi dans le groupe constitué par un copolymère d'acrylate, un copolymère ou terpolymère à base de méth(acrylique), un copolymère ou terpolymère à base d'acide maléique, un dérivé de p-tert-octylphénol avec une chaîne latérale de polyéthylène glycol, un ester d'acide gras de sorbitane, un ester d'acide gras de polyoxyéthylène sorbitane, un copolymère de poly(3,4-dihydroxyphénylalanine)-polylactide et un copolymère de poly(3,4-dihydroxyphénylalanine)-polyéthylène glycol.

2. Procédé selon la revendication 1,
**caractérisé en ce que**
l'agent d'extraction est une solution aqueuse.

3. Procédé selon la revendication 1 ou 2,
**caractérisé en ce que**
l'agent d'extraction contient de l'acide chlorhydrique ou de l'acide sulfurique.

4. Procédé selon la revendication 3,
**caractérisé en ce que**
l'agent d'extraction contient l'acide chlorhydrique à une concentration de 1,5 à 6 mol/l, de préférence de 1,8 à 4 mol/l, ou contient l'acide sulfurique à une concentration de 0,1 à 3 mol/l, de préférence de 0,3 à 1 mol/l.

5. Procédé selon l'une des revendications 1 à 4,
**caractérisé en ce que**
une suspension qui s'est formée par l'extraction à partir de l'agent d'extraction, chargé de composants de l'acier décomposé, et des particules est soumise, le cas échéant plusieurs fois, à un procédé de séparation, de préférence à une centrifugation ou/et à une sédimentation.

6. Procédé selon la revendication 5,
**caractérisé en ce que**
lors de la mise en oeuvre du procédé de séparation, l'agent d'extraction chargé est au moins partiellement séparé des particules.

7. Procédé selon la revendication 6,
**caractérisé en ce que**
après la séparation partielle de l'agent d'extraction chargé des particules, un solvant, de préférence de l'eau, est ajouté à une suspension résiduelle formée par l'agent d'extraction, restant après la séparation, et par les particules.

8. Procédé selon l'une des revendications 5 à 7,
**caractérisé en ce que**
le procédé de séparation est mis en oeuvre de telle sorte que l'agent dispersant qui s'est placé sur les particules lors de l'extraction reste sur les particules pour stabiliser la suspension.

9. Procédé selon l'une des revendications 5 à 8,
**caractérisé en ce que**
une analyse des particules, en particulier une mesure de la taille des particules ou de la distribution des tailles de particules, est effectuée directement sur la suspension obtenue après le procédé de séparation ou sur un échantillon qui a été préparé directement à partir de la suspension, de préférence par séchage.

10. Procédé selon l'une des revendications 1 à 9,
**caractérisé en ce que**
les particules sont analysées par diffusion dynamique de la lumière, par fractionnement champ-flux asymétrique, par ultracentrifugation analytique ou/et par microscopie électronique, de préférence par microscopie électronique en transmission (MET) ou par microscopie électronique en transmission à balayage (METS).

11. Procédé selon l'une des revendications 1 à 10,
**caractérisé en ce que**
l'acier est un acier micro-allié, et les particules contiennent les éléments d'alliage titane, niobium, aluminium et/ou vanadium, de préférence du nitrure de titane, du carbure de titane, du carbonitrure de titane, du carbure de niobium, du carbonitrure de niobium, et/ou des carbures, nitrures ou carbonitrures comprenant du titane et du niobium.

12. Suspension, contenant un agent d'extraction de particules, en particulier de précipités ou/et d'inclusions, dans l'acier, agent qui contient un acide décomposant l'acier, des particules extraites de l'acier, et des composants de l'acier, décomposés par l'extraction,
**caractérisée en ce que**
pour éviter l'agglomération des particules, l'agent d'extraction contient un agent dispersant qui contient un copolymère choisi dans le groupe constitué par un copolymère d'acrylate, un copolymère ou terpolymère à base de méth(acrylique), un copolymère ou terpolymère à base d'acide maléique, un dérivé de p-tert-octylphénol avec une chaîne latérale de polyéthylène glycol, un ester d'acide gras de sorbitane, un ester d'acide gras de polyoxyéthylène sorbitane, un copolymère de poly(3,4-dihydroxyphénylalanine)-polylactide et un copolymère de poly(3,4-dihydroxyphénylalanine)-polyéthylène glycol.
